# EUROPÄISCHE PATENTSCHRIFT

(11) **EP 0 569 583 B1**
(45) Veröffentlichungstag und Bekanntmachung des Hinweises auf die Patenterteilung: **01.08.2001**
(21) Anmeldenummer: 93901669.7
(22) Anmeldetag: 02.12.1992
(51) Int. Cl.: A61K 51/00

(54) **KONTROLLIERTER EINSATZ VON POLYETHERSUBSTITUIERTEN TUMORMITTELN IN DIAGNOSE UND THERAPIE**
CONTROLLED USE OF POLYETHER-SUBSTITUTED TUMOR AGENTS IN DIAGNOSIS AND THERAPY
UTILISATION CONTROLEE D'AGENTS TUMORAUX A SUBSTITUTION POLYETHER EN DIAGNOSTIC ET EN THERAPIE

(30) Priorität: 02.12.1991 DE 4139715
(43) Veröffentlichungstag der Anmeldung: 18.11.1993
(73) Patentinhaber: Deutsches Krebsforschungszentrum Stiftung des öffentlichen Rechts, 69120 Heidelberg (DE)
(72) Erfinder: SINN, Hans-J., D-6908 Wiesloch (DE); SCHRENK, Hans-Hermann, D-6721 Zeiskam (DE); MAIER-BORST, Wolfgang, D-6901 Dossenheim (DE); FRIEDRICH, Eckhard, D-6741 Ilbesheim (DE); GRASCHEW, Georgi, D-13129 BERLIN (DE)
(74) Vertreter: Schüssler, Andrea, Dr.
(86) Internationale Anmeldenummer: EP9202789
(87) Internationale Veröffentlichungsnummer: WO9310743

(56) Entgegenhaltungen:
- EP-A- 0 014 263
- EP-A- 0 207 557
- EP-A- 0 336 879
- DE-A- 4 017 439
- STN FILE SUPPLIER & FILE CA (KARLSRUHE) & CHEMICAL ABSTRACTS, vol. 109, no.1 Columbus Ohio,US; abstract no.6551w, "PORPHYRIN DERIVATIVES FOR DIAGNOSIS AND PHOTODYNAMIC THERAPY OF TUMORS AND A PROCESS FOR THEIR PREPARATION"
- STN FILE SUPPLIER & FILE CA & CHEMICAL ABSTRACTS, vol. 112, no.3 Columbus, Ohio, US; abstract no. 21146y, "PREPARATION OF CYCLOTRIPHOSPAZENE DERIVATIVES BOUND TO HYDROPHILIC POLYMER AND THERAPEUTIC PHYSIOLOGICALLY ACTIVE SUBSTANCE"
- STN FILE SUPPLIER & FILE CA (KARLSRUHE) & CHEMICAL ABSTRACTS, vol. 109, no.1 Columbus Ohio,US; abstract no.6551w, "PORPHYRIN DERIVATIVES FOR DIAGNOSIS AND PHOTODYNAMIC THERAPY OF TUMORS AND A PROCESS FOR THEIR PREPARATION"
- STN FILE SUPPLIER & FILE CA & CHEMICAL ABSTRACTS, vol. 112, no.3 Columbus, Ohio, US; abstract no. 21146y, "PREPARATION OF CYCLOTRIPHOSPAZENE DERIVATIVES BOUND TO HYDROPHILIC POLYMER AND THERAPEUTIC PHYSIOLOGICALLY ACTIVE SUBSTANCE"

## Beschreibung

Die Erfindung ist eine weitere Ausgestaltung der Patentanmeldung DE 40 17 439 und betrifft die Verwendung von polyethersubstituierten Tumormitteln zum kontrollierten Einsatz in Diagnose und Therapie.

Die Patentanmeldung DE 40 17 439 bezieht sich auf tumoraktive bzw. Tumordiagnostik-Substanzen mit bevorzugter Anreicherung im Tumor und mit mindestens zwei phenolischen Hydroxylgruppen oder mindestens einer aliphatischen Aminogruppe, bei denen mindestens zwei phenolische Hydroxylgruppen oder mindestens eine aliphatische Aminogruppe mit einer Polyethylenglykolkette vom Polymerisationsgrad n = 5 bis 250 substituiert ist, deren endständige Hydroxylgruppe durch C₁ - C₁₂-Alkylester oder /-ether substituiert ist. Diese Substanzen können radioaktiv markiert sein.

Die Anreicherung, die sich insbesondere bei den radioaktiv markierten Substanzen deutlich in den Aufnahmen zeigt, ist außerordentlich überraschend. Es besteht nun die Möglichkeit, Substanzen insbesondere für die Diagnostik und die Therapie von Tumoren, gezielt an Ort und Stelle zu bringen.

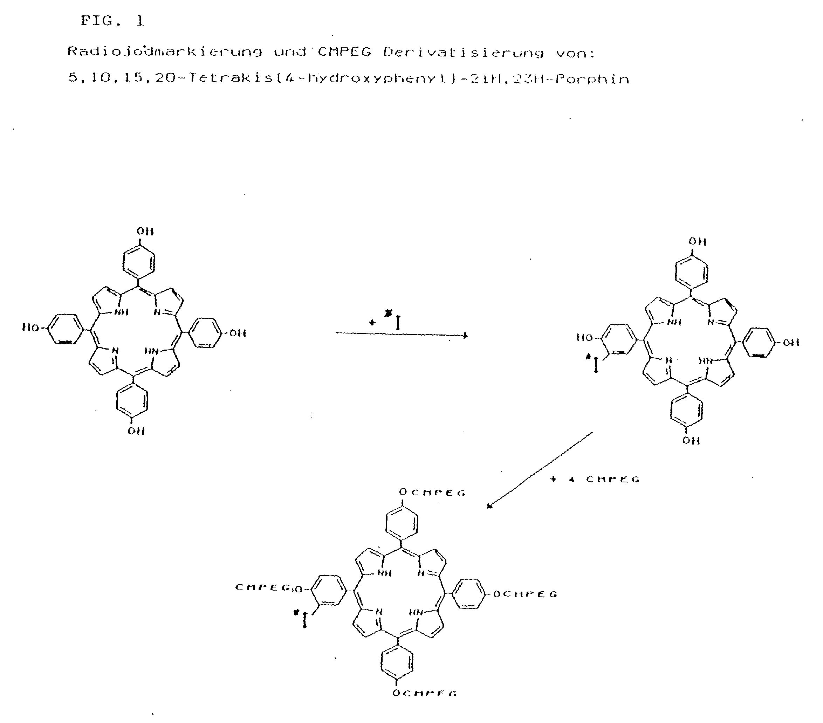

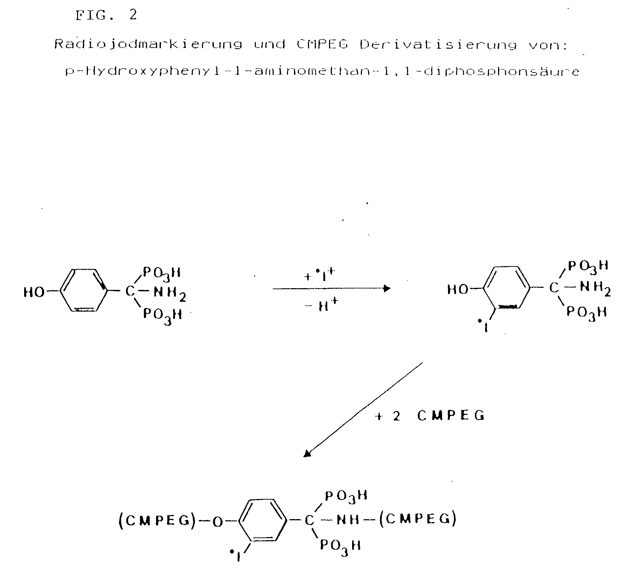

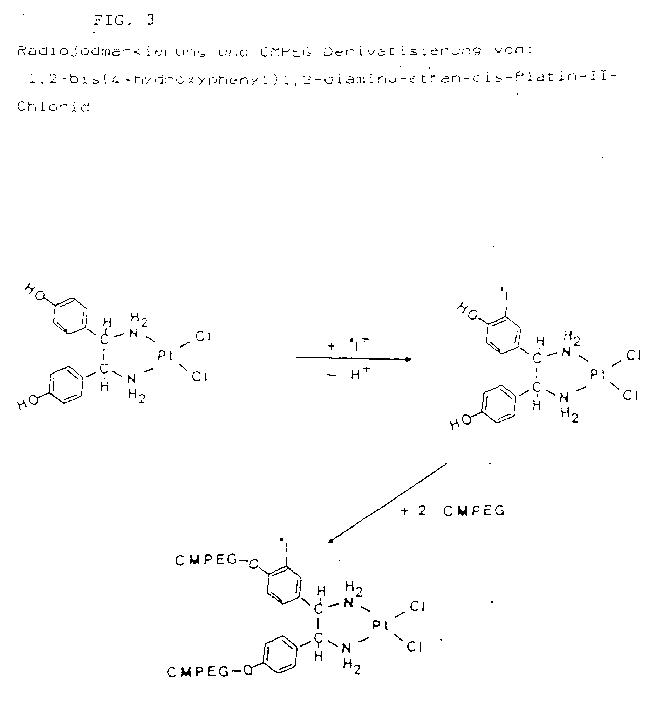

Beispiele für polyetherderivatisierende Substanzen, die alle eine hohe spezifische Anreicherung im Tumorgewebe aufweisen und eine wesentlich reduzierte Belastung von Normalgeweben bzw. Organen zur Folge haben, sowie deren Einsatzmöglichkeiten sind die folgenden:
1. Porphine und Phthalocyanine, die vom Typ her bekannt sind, für die laserinduzierte Fluoreszenzdiagnostik und photodynamische Therapie (PDT) von Tumoren. Als radiojodmarkierte Verbindung zur nuklearmedizinischen Lokalisation unbekannter Metastasen, zur Bestimmung des optimalen Zeitpunkts für eine PDT sowie für eine mögliche Radiojodtherapie. Reaktionschemata für die Radiojodmarkierung sowie CMPEG-Derivatisierung sind in **Fig.** 1 für 5,10,15,20-tetrakis(4-Hydroxyphenyl)-21H, 23H-Porphin gezeigt
2. Radiojodmarkierte Diphosphonate zur nuklearmedizinischen Lokalisation von Tumoren und/oder Metastasen bzw. deren Radiojodtherapie. Reaktionsschemata für die Radiojodmarkierung sowie CMPEG-Derivatisierung sind in **Fig.** 2 für p-Hydroxyphenyl-1-aminomethan-1,1-diphosphorsäure gezeigt.
3. Cis-Platin und cis-Platin-Abkömmlinge, Tetracycline und Stereoide sowie ähnliche Verbindungen für die Chemotherapie. Radiojodmarkierte Produkte dienen zur nuklearmedizinischen Erfassung der individuellen Substanzverteilungsmuster, Reaktionsschemata für die Radiojodmarkierung sowie CMPEG-Derivatisierung sind in **Fig.** 3 für 1,2-bis/4-Hydroxyphenyl)1,2-diamino-ethan-cis-Platin-II-Chlorid gezeigt
4. Mit Radiojod, Radiobrom oder mit radioaktiven Metallionen markierte Peptide, Porphine, Phthalocyanine und ähnliche Verbindungen zur positiven Darstellung von Tumoren mit planar bzw. tomographisch abbildenden gamma-Kamera.
5. Hoch jodierte Verbindungen wie z. B.: Porphine, oligomere Tyrosine usw. zur positiven Darstellung von Tumoren mit Hilfe der Computer-Tomographie (CT).
6. Metallkomplexe wie z. B.: Gadolinium-Phthalocyanine zur positiven Tumordarstellung mit Hilfe der Magnet-Resonanz-Tomographie (MRT).
7. Radiosensibilisatoren wie z. B. C. Nitroimidazol als unterstützende Maßnahme bei der Strahlentherapie von Tumoren mit externen oder tumorinkorporierten Strahlenquellen z. B. bei der PDT, die auch schon unter Punkt 1 erörtert ist.
8. Luminole zur intratumoralen Erzeugung von Chemilumineszenz und ihre Anwendung zur direkten Phototherapie neoplastischer Gewebe.
9. Borsäureester für die Neutroneneinfangtherapie von Tumoren.
10. Dysprosium 164 für Phthalocyanine (nicht z. B. für Porphyrine, da Dy dafür zu groß ist). Dy¹⁶⁴, das letzte stabile Isotop, fängt thermische Neutronen ein und emittiert β, wobei es in Dy¹⁶⁴ übergeht.

Die Polyethylenglykolreste sind derart, daß die Gesamtverbindungen ein Molekulargewicht von 2000 bis 10 000 oder darüber haben. Nachdem mindestens zwei solcher Reste an das Cytostatikum gebunden werden sollen, bedeutet dies ein Mindestmolekulargewicht für die z. B. Methoxypolyethylenglykole von knapp 1000. Besonders bevorzugt ist ein Molekulargewicht von etwa 5000. Bei diesen Polyethylenoxidresten ist ein endständiges Hydroxyl durch eine C₁-C₁₂-Alkylether- oder -estergruppe substituiert. Die Methoxyverbindung ist dabei bevorzugt. Methoxypolyethylenoxide mit einem Molekulargewicht von etwa 5000 sind im Handel erhältlich und können, evtl. nach üblicher bekannter Reinigung, direkt für die Synthese der erfindungsgemäßen Derivate von Cytostatika verwendet werden. Wenn ein solches Mittel z. B. mit Jod markiert werden soll, dann wird zuerst das Jodatom eingeführt und dann wird, wie üblich, mit einem Linker, vorzugsweise Cyanurchlorid, die Polyoxyethylenkette angefügt.

Das Gesamtmolekulargewicht der erfindungsgemäßen Derivate von Cytostatika beträgt vorzugsweise mehr als 10 000. Wenn man z. B. ein Porphin gemäß **Fig.** 1 verwendet, dessen Molekulargewicht ca. 678 beträgt, so werden hier 4 Methoxypolyethylenoxidreste mit einem Molekulargewicht von etwa 5000, jedoch mindestens etwa 2500 angeheftet.

Bei der Verbindung gemäß **Fig.** 2 der gezeigten Diphosphorsäureverbindung sollten daher zwei Methoxypolyethylenoxidreste vom Molekulargewicht etwa 5000 oder noch besser 6000 angeheftet werden.

Wie erwähnt, gewährt diese Polyethylenglykolsubstitution durch die Kettenlänge und/oder die Verzweigung der Kette die Bildung eines Gesamtblockes mit Serumalbumin, dessen Größe die Ausschlußgrenze der Niere übersteigt

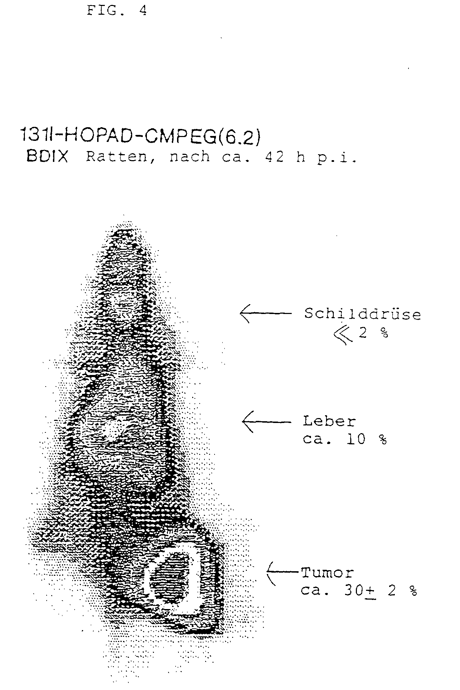

, Die Konzentration des verabreichten Mittels, wenn dieses ein Phthalocyanin ist, ist im Tumor so hoch, daß man den Tumor blaugefärbt sieht. Interessant ist, daß der Farbstoff nur in vitalem Tumorgewebe abgelagert wird, nicht jedoch im abgestorbenen Gewebe, wie dies die beigefügte **Fig.** 4 zeigt.

Die folgenden Beispiele erläutern die Erfindung und zeigen die Herstellung der derivatisierten Cytostatika:

### Beispiel 1

### Herstellung von radiojodmarkiertem und CMPEG-derivatisiertem TOP

Zu 100 µg Tetra-(4-hydroxyphenyl)-Porphin (TOP), gelöst in 100 µg Dimethylacetamid, gibt man 5-20 µl einer alkalischen Lösung (pH 8-11) von Radiojod (Jodid) mit einer Aktivität von 18,5 MBq (500 µCi) bis 185 MBq (5 mCi) und 3-10 µg N-Chlorsuccinimid (NClS), gelöst in 2-5 µl Acetonitril. Nach einer Reaktionszeit von 20-30 min fügt man 4 mg alpha-Dichlorotriazin-Ω-methoxy-polyethylenglykol (n =100-110, also 100-110 Ethylenglykoleinheiten) gelöst in 40 µl Dioxan und 100 µl 0,17 M Natriumbicarbonatlösung hinzu. Nach einer Reaktionszeit von 20-30 min wird das Reaktionsgemisch mit dest. Wasser auf 200 µl aufgefüllt und die organischen Lösungsmittel über eine Ultrafiltrationseinheit mit einer Ausschlußgrenze von 5 kD abgetrennt. Es ist zu beachten, daß als Lösungsmittel ein organisches Lösungsmittel mit hoher DK verwendet wird
Analytik:
Dünnschichtchromatographie:
Platten:
Kieselgel 60 (5 × 20 cm) (ohne Fluoreszenzindikator)
Laufmittel: Methanol, 10% NH₄acetat in Wasser (1/1)
Laufstrecke: 10 cm
Rf-Werte:
TOP 0,95-0,98
TOP-CMPEG 0,0

### Beispiel 2

### ¹³¹I-Markierung und CMPEG-Derivatisierung von: p-Hydroxyphenyl-1-aminomethan-1,1-diphosphonsäure (HOPAD)

Zu 100 µl einer Lösung von HOPAD in 0,17 M NaHCO₃ (1 mg/ml) fügt man 2-5 mCi (74-185 MBq) einer ¹³¹I-Na-Jodid-Lösung (10-25 µl) und 3-7,5 µg N-Bromosuccinimid (NBS), gelöst in 0,17 M NaHCO₃ (1 mg/ml) hinzu. Nach einer Reaktionszeit von 10 min bei Raumtemperatur beträgt die durchschnittliche ¹³¹I-Einbaurate 98%. Man fügt nun in 1,4-Dioxan gelöstes CMPEG (n = 100-110)(100 mg/ml) in einem molaren Verhältnis von 3:1 (etwa 5,5 mg) hinzu. Nach einer Reaktionszeit von etwa 30 min wird nicht gebundenes ¹³¹I und nicht derivatisiertes HOPAD durch Ultrafiltration oder Dialyse abgetrennt.

Es wurden folgende Verbindungen untersucht:

Abkürzungen:
TOP = Tetra-(hydroxyphenyl)-porphin
TOPPC = Tetra-(hydroxy-phenoxy)-phthalocyanin
HOPAD = Hydroxyphenyl-aminomethan-diphosphonat
HPBT= Hematoporphyrin-bis-tyrimid
CMPEG = alpha-dichloro-triazin-Ω-methoxy-polyethylenglykol (n = 100-110)
AMPEG = alpha-amino-Ω-methoxy-polyethylenglykol (n = 100 - 110)

In der gleichen Weise wie hier in Beispiel 1) a ¹³¹I-Tetra(hydroxyphenyl)-porphin-(CMPEG)₄ gezeigt, wurden noch hergestellt:
1)b ¹¹¹-In-Tetra-(hydroxyphenyl)-porphin-(CMPEG)₄
1)c ¹¹¹-In-Tetra-(hydroxyphenoxy)-phthalocyanin-(CMPEG)₄,
1)d ¹³¹-I-Tetra-(hydroxyphenoxy)-phthalocyanin-(DMPEG)₄,
1)e ¹³¹-I-Hematoporphyrin-bis-tyrimid-(CMPEG)₂ und
1)f ¹³¹-I-Hydroxyphenylaminomethandiphosphat-(CMPEG)₂,
die alle Anwendung in der Nuklearmedizin finden.

Für die Anwendung in der Fluoreszenzdiagnostik und der photodynamischen Therapie wurde in entsprechender Weise hergestellt:
2a) TOP-(CMPEG)₄
2b) TOPPC-(CMPEG)₄
2d) HPBT-(CMPEG)₂

Die folgenden NMR-Untersuchungen wurden ebenfalls in entsprechender Weise hergestellt.
3a) MN-TOP-(CMPEG)₄
3b) Gd-TOPPC-(CMPEG)₄

Schließlich wurden für CT-Untersuchungen die folgenden Verbindungen gemäß Beispiel 2 hergestellt.
4a) Tri-jod-phenol-CMPEG
4b) Tri-jod-benzoesäure-AMPEG
4c) Iodoxaminsäure-(AMPEG)₂

Die Konzentrierung dieser Mittel wurde an folgenden experimentellen Tumoren und an folgenden Tiermodellen geprüft:

| Tumoren | Tiermodell |
|---|---|
| a) experimentelle Tumoren | |
| Ovarialcarcinom (O-342) | Ratte, BD IX |
| Walker Carcinosarcom | Ratte, Wistar |
| Yoshida Hepatom | Ratte, Wistar |
| Novikoff Hepatom | Ratte, Wistar |
| Osteosarcom | Ratte, SD curly |

| b) Xenotransplantate, human | Nacktmaus |
|---|---|
| CX 1 : Coloncarcinom | Nacktmaus |
| LX 1 : axillare Lymphknotenmetastase von einem Lungencarcinom | Nacktmaus |
| MX 1 : Mamacarcinom | Nacktmaus |
| HS 1 : Osteosarcom | Nacktmaus |
| MR I-H-186: Carcinom corpus uteri | Nacktmaus |
| HeLa : Cervixcarcinom | Nacktmaus |
| EI 28 : Blasencarcinom | Nacktmaus |
| GXF-96 : Magencarcinom | Nacktmaus |

Alle diese experimentellen Tumoren und Xenotransplantate wurden mit den Verbindungen a und c gemäß Beispiel 1 (also 1a und 1c) und drei davon, nämlich das Walker Carcinosarcom, das Osteosarcom und das Ovarialcarcinom (O-342) auch mit dem Diphosphat gemäß Beispiel 2 untersucht.

Bei allen diesen Anwendungsversuchen wurden immer 300 µC = 11,1 MBq verabreicht Es zeigten sich mit den eingesetzten Mitteln, nämlich 1a und 1c und die Verbindung aus Beispiel 2 eine Konzentration von mindestens 30% des Mittels an der gewünschten Stelle, also am Tumor. Die beigefügte **Fig.** 4 zeigt das Bild einer BDIX-Ratte mit Ovarialcarcinom 42 h nach Injektion, die mit ¹³¹J-HOPAD-CMPEG, also ¹³¹J-Hydroxyphenyl-1-aminome-than-1,1-diphosphonat-Cyanurchlorid aktiviertes Polyethylenglykol (n=ca. 100 bis 110) in einer Menge von 300 µC behandelt war. Das Bild zeigt, daß ganz unten, im Tumor ca. 30+/-2% des Mittels konzentriert sind, während in der Mitte die Leber ca. 10% zeigt und die Schilddrüse, die oberhalb der Leber sichtbar ist, weit weniger als 2% des Mittels aufgenommen hat Diese %-Angaben beziehen sich auf die Gesamtmenge an die mit radiojodmarkierten Mittel im Gesamtkörper der Ratte.

Normal ist bei Verabreichung üblicher Cytostatika, also von Cytostatika, die nicht mit Polyethylenglykolderivat derivatisiert sind, eine Konzentration im Tumor, die etwa so hoch ist, wie sie hier in der Schilddrüse auftritt, d.h. 0,05 bis 0,5% der gesamten applizierten Menge, wobei jedoch bis zu 90% der verabreichten Menge in der Leber vorliegen, was natürlich zu einer enormen Belastung dieses Organs führt, eine Belastung, die nicht nur unerwünscht, sondern auch ausgesprochen schädlich ist.

Die Derivatisierung üblicher Cytostatika, die mindestens 2 phenolische Hydroxylgruppen enthalten oder eine aliphatische Aminogruppe aufweisen, mit höher molekularen Polyoxyethylenglykolen mit blockierter Endgruppe führt somit zu einer wesentlichen Konzentrierung des Mittels im vitalen Tumorgewebe und somit zu einer wesentlich besseren Wirkung am gewünschten Ort und einer wesentlichen Entlastung von Leber und Schilddrüse, was natürlich die Nebenwirkungen solcher Cytostatika bedeutend herabsetzt

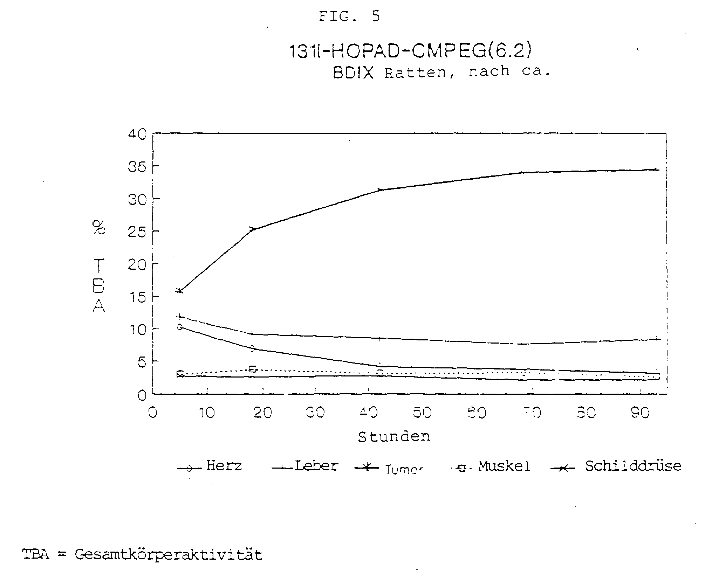

Die beigefügte **Fig.** 5 zeigt die Messung einer mit ¹³¹I-TOP-(CMPEG)4 behandelten BD IX Ratte (auch hier Ovarialcarcinom). Es zeigt sich ein ähnliches Ergebnis wie das von **Fig.** 4.

Von der gesamten Körperaktivität an radioaktivem Jod finden sich nach 150 h etwa 23% im Tumor, etwa 12% in der Leber, etwa 5% im Blut und etwa 3-4% jeweils in den Muskeln und in der Schilddrüse. Der Rest ist natürlich, wie auch bei der Aufnahme von Fig. 4, über den restlichen Körper verteilt.

Auch hier ist eine starke Konzentration im Tumor und eine wesentliche Entlastung der Leber und der Schilddrüse festzustellen, da auch bei dieser nicht derivatisierten Verbindung gilt, da auch bei deren Verabreichung höchstens soviel im Tumor enthalten ist, wie in der Schilddrüse.

Es hat sich nun gezeigt, daß obige, radioaktiv-markierte Substanzen, insbesondere dann, wenn die Polyethylenglykolketten über biologisch nicht- oder nur schwer-hydrolysierbare Linker, beispielsweise aus Cyanurchlorid, angefügt sind, bestens zum kontrollierten Einsatz in Diagnose und Therapie geeignet sind. Insbesondere sind folgende Vorteile bei ihrer Verwendung anzuführen:
a) Durch den Einsatz diagnostischer Mengen der entsprechenden Substanz (z.B. 100-200 µg und weniger als 74 MBq (2 mCi) eines gebräuchlichen Radionuklids, beispielsweise ¹³¹J, eventuell mit einem Zuschlag an ¹²⁵J oder ⁷⁷Br als Einheit konfektioniert) kann mittels Anwendung nuklearmedizinischer Untersuchungstechniken geklärt werden, ob die entsprechende Substanz vom Tumor überhaupt aufgenommen wird und wenn ja, wieviel, ohne dem gesunden Organismus eine nennenswerte Belastung zuzumuten. Mit diesem Ergebnis kann individuell für jeden Patienten die Dosis ermittelt werden, die maximal erforderlich ist, um eine zuverlässige Diagnose und erfolgreiche Therapie erwarten zu können, ohne durch Überdosierungen den Restorganismus unnötig zu belasten.
b) Durch das nuklearmedizinische "follow up" ist während der Therapie eine Quantifizierung der vom Tumor aufgenommenen Substanzmenge möglich. Im Falle der photodynamischen Therapie führt dies dazu, daß die Bestrahlung mit Laserlicht erstmalig individuell zum optimalen Zeitpunkt realisiert werden kann.
c) Bei einer zweiten Behandlung kann mit Hilfe der nuklearmedizinischen Untersuchungstechnik ermittelt werden, ob sich das Speicherverhalten der jeweiligen Substanz gegenüber der Erstbehandlung geändert hat, was bisher bei keiner der gängigen Substanzen möglich war.
d) Speziell im Falle der photodynamischen Therapie zeigt eine rasche Aktivitätsabnahme im Tumorbereich im Zeitraum von 24 h nach erfolgter Laserlichtbestrahlung eine erfolgreiche Zerstörung von vitalem Tumorgewebe an, während ein mehr oder weniger konstant bleibender Aktivitätspegel im Tumorbereich anzeigt, daß die Bestrahlung unter geänderten Bedingungen wiederholt werden muß.
e) Selbst bei dem Einsatz geringfügiger Mengen an ¹³¹J, die für die diagnostischen Maßnahmen erforderlich sind, erfährt der Tumor infolge der hohen Anreicherungen eine zusätzliche nicht zu vernachlässigende Strahlenbelastung, die dem kurativen Effekt der Maßnahme zuträglich ist.

Insgesamt gesehen, kann also mit den in Patentanmeldung DE 40 17 439 beschriebenen Substanzen eine Tumor-Diagnose und -Therapie durchgeführt werden, in der jeweils der Zustand und die Entwicklung des Tumors individuell bei jedem Patienten berücksichtigt werden kann, indem nur in solchen Mengen konfektionierte Mittel verwendet werden, wie sie normalerweise für die Diagnostik eingesetzt werden.

## Patentansprüche

1. Verwendung von radioaktiv markierten tumoraktiven bzw. Tumordiagnostik-Substanzen mit bevorzugter Anreicherung im Tumor und mit mindestens zwei phenolischen Hydroxylgruppen oder mindestens einer aliphatischen Aminogruppe, bei denen mindestens zwei phenolische Hydroxylgruppen oder mindestens eine aliphatische Aminogruppe mit einer Polyethylenglykolkette vom Polymerisationsgrad n = 5 bis 250 substitutiert ist, deren endständige Hydroxylgruppe durch C₁ - C₁₂-Alkylester oder /-ether substitutiert ist zur Herstellung eines Arzneimittels, das 100-200 µg der mit weniger als 74 MBq (2mCi) radioaktiv markierten tumoraktiven bzw. Tumordiagnostik - Substanzen aufweist, zur nuklearmedizinischen Charakterisierung eines Tumors.

2. Verwendung nach Anspruch 1, dadurch gekennzeichnet, daß in den Tumormitteln die Polyethylenglykolketten über biologisch nicht- oder nur schwer-hydrolysierbare Linker angefügt sind.

3. Verwendung nach Anspruch 2, dadurch gekennzeichnet, daß zur Herstellung des biologisch nicht-hydrolysierbaren Linkers Cyanurchlorid verwendet wird.

## Claims

1. Use of radioactively labeled tumor-active or tumor-diagnostic substances with preferred concentration in the tumor and at least two phenolic hydroxyl groups or at least one aliphatic amino group, wherein at least two phenolic hydroxyl groups or at least one aliphatic amino groups is substituted with a polyethylene glycol chain having a polymerization degree of n = 5 to 250, whose terminal hydroxyl group is substituted by C₁-C₁₂ alkyl ester or ether for the production of a medicament having 100 - 200 µg of the tumor-active or tumor-diagnostic substances radioactively labeled with less than 774 MBq (2 mCi) for the nuclear medical characterization of a tumor.

2. Use according to claim 1, characterized in that the polyethylene glycol chains in the tumor agents are attached by means of linkers which are not biologically hydrolyzable or hydrolyzable only with difficulty.

3. Use according to claim 2, characterized in that cyanuric chloride is used for the production of the biologically non-hydrolyzable linker.

## Revendications

1. Utilisation de substances marquées radioactivement, actives dans des tumeurs ou pour le diagnostic de tumeurs, avec une concentration préférée dans la tumeur et avec au moins deux groupes hydroxyle phénoliques ou au moins un groupe amino aliphatique, dans lesquelles au moins deux groupes hydroxyle phénoliques ou au moins un groupe amino aliphatique est/sont substitué(s) par une chaîne de polyéthylèneglycol ayant un degré de polymérisation n = 5 à 250, dont le groupe hydroxyle terminal est substitué par un ester ou un éther d'alkyle en C₁-C₁₂, pour la fabrication d'un médicament qui contient de 100 à 200 µg des substances marquées radioactivement avec moins de 74 MBq (2mCi), actives dans des tumeurs ou pour le diagnostic de tumeurs, pour la caractérisation d'une tumeur en médecine nucléaire.

2. Utilisation selon la revendication 1, caractérisée en ce que, dans les agents antitumoraux, les chaînes de polyéthylèneglycol sont ajoutées au moyen de segments de liaison biologiquement non hydrolysables ou seulement difficilement hydrolysables.

3. Utilisation selon la revendication 2, caractérisée en ce qu'on utilise du chlorure de cyanuryle pour la préparation du segment de liaison biologiquement non hydrolysable.
